Europäisches Patentamt

(19) European Patent Office

Office européen des brevets

(11) Numéro de publication : **0 330 567 B1**

## (12) FASCICULE DE BREVET EUROPEEN

(45) Date de publication du fascicule du brevet :
23.10.91 Bulletin 91/43

(51) Int. Cl.⁵ : **A61K 35/78**

(21) Numéro de dépôt : **89400495.1**

(22) Date de dépôt : **22.02.89**

(54) Procédé d'obtention d'un extrait de feuilles de ginkgo biloba.

(30) Priorité : **24.02.88 FR 8802227**

(43) Date de publication de la demande :
**30.08.89 Bulletin 89/35**

(45) Mention de la délivrance du brevet :
**23.10.91 Bulletin 91/43**

(84) Etats contractants désignés :
**AT BE CH DE ES GB GR IT LI LU NL SE**

(56) Documents cités :
EP-A- 0 086 315
EP-A- 0 237 066
FR-A- 2 007 352
FR-A- 2 132 761
CHEMICAL ABSTRACTS, vol. 101, no. 8, 20 août 1984, page 306, no. 60122e, Columbus, Ohio, US; & JP-A-58 210 034 (KURARAY CO. LTD) 07-12-1983

(56) Documents cités :
CHEMICAL ABSTRACTS, vol. 107, no. 4, 27 juillet 1987, page 324, no. 28395j, Columbus, Ohio, US; & JP-A-62 33 118 (KURARAY CO. LTD) 13-02-1987

(73) Titulaire : **PIERRE FABRE SANTE**
**45, Place Abel-Gance**
**F-92100 Boulogne (FR)**

(72) Inventeur : **Ayroles, Georges**
**99, Chemin de Lapeyre**
**F-81600 Gaillac (FR)**
Inventeur : **Rossard, René-Marc**
**Chemin des Aubaresses**
**F-81600 Gaillac (FR)**
Inventeur : **Cadiou, Michel**
**La Berlandié Montels**
**F-81140 Castelnau-de-Montmiral (FR)**

(74) Mandataire : **Warcoin, Jacques et al**
**Cabinet Régimbeau 26, avenue Kléber**
**F-75116 Paris (FR)**

## Description

La présente invention concerne un procédé d'obtention d'un extrait de feuilles de Ginkgo biloba destiné à l'administration par voie orale ou par voie injectable pour traiter les troubles de la circulation artérielle, capillaire et veineuse.

Les extraits de feuilles de Ginkgo biloba sont utilisés depuis très longtemps dans le traitement des troubles de la circulation périphérique et cérébrale, en particulier chez les personnes âgées. On connaît également la nature des substances actives contenues dans ces extraits, qui sont responsables de leur activité veinotrope (H. PETER, J. FISEL et W. WEISSER : Pharmacologie des principes actifs de Ginkgo biloba, pages 719-725, n° 6, 1966).

On sait également depuis longtemps que les extraits de feuilles vertes de Ginkgo biloba renferment certains constituants qui posent différents problèmes, notamment de toxicité, lorsque ces extraits sont destinés à être administrés par voie injectable. Il s'agit de dérivés polyphénoliques, généralement désignés sous le nom de proanthocyanidines, en particulier des prodelphinidines.

Ces problèmes sont notamment relatés dans les publications suivantes :
— R. HEMMER Arzneimittel Vorschung 17, 491 (1967)
— K. WEINGES Arzneimittel Vorschung 18, 539 (1968)
— K. WEINGES Arzneimittel Vorschung 19, 328 (1969).

Différentes solutions ont été préconisées à ce jour pour obtenir un extrait de Ginkgo biloba faiblement dosé en proanthocyanidines. Cependant, aucune de ces solutions ne donnent entière satisfaction, soit parce que le taux des proanthocyanidines reste trop élevé dans l'extrait final, soit encore parce que la purification entraîne des difficultés de mise en oeuvre, notamment consécutives à l'utilisation de solvants, polymères ou dérivés de métaux lourds généralement toxiques. Au surplus, il a été observé qu'une élimination poussée des proanthocyanidines entraînait généralement une diminution concomitante des substances actives de l'extrait de Ginkgo biloba, en particulier des glucosides flavoniques, de la quercétine et du kaempférol. Enfin, le rendement d'extraction était très défavorablement influencé par les traitements connus d'élimination des proanthocyanidines.

La présente invention vise précisément à pallier tous ces inconvénients, en fournissant un procédé d'obtention d'un extrait de feuilles de Ginkgo biloba qui, au surplus, s'avère plus simple à mettre en oeuvre et plus économique.

Conformément à la présente invention, le procédé d'obtention d'extrait de feuilles de Ginkgo biloba est caractérisé en ce que l'on effectue les opérations successives suivantes :
— broyage des feuilles de Ginkgo biloba,
— extraction du broyat de feuilles de Ginkgo biloba à l'aide d'un solvant cétonique aqueux,
— concentration des jus d'extraction afin de précipiter les biflavonoïdes et les substances hydrophobes,
— filtration du concentrat aqueux,
— basification du filtrat pour faire précipiter les proanthocyanidines,
— élimination par filtration de la fraction insoluble contenant les proanthocyanidines,
— acidification du filtrat,
— extraction liquide-liquide du filtrat par une cétone en $C_4$-$C_6$ en présence de sulfate d'ammonium, et
— récupération de l'extrait par mise à sec de la phase cétonique.

Conformément à ce procédé, les proanthocyanidines sont éliminées par précipitation suite à une basification du filtrat aqueux, suivie d'une filtration de la fraction insoluble. Cette basification du filtrat est avantageusement réalisée jusqu'à obtention d'une valeur de pH supérieure à 8 et pouvant même atteindre des valeurs beaucoup plus élevées voisines de 14.

Les proanthocyanidines peuvent être précipitées par basification du filtrat aqueux au moyen d'ammoniaque, d'ammoniac, ou d'autres agents basiques, tels que les hydroxydes de métaux alcalins ou alcalino-terreux, notamment les hydroxydes de sodium et de calcium, ou encore les amines, notamment les alkylamines.

Après élimination des proanthocyanidines, il faut procéder à une acidification du filtrat, de façon à se placer à nouveau dans de bonnes conditions pour procéder à l'extraction liquide-liquide subséquente. Cette acidification est obtenue de façon satisfaisante dans la pratique par addition d'acide sulfurique. Le pH du filtrat peut être ainsi abaissé jusqu'à une valeur voisine de 1.

Selon une autre caractéristique additionnelle de la présente invention, après acidification le filtrat est soumis à une extraction liquide-liquide, en présence de sulfate d'ammonium. A titre d'exemples de cétones en $C_4$-$C_6$ utilisées pour la mise en oeuvre de cette extraction liquide-liquide, on mentionnera la méthyléthylcétone et la méthylisobutylcétone. Ces cétones peuvent être utilisées en mélange avec de l'acétone jusqu'à atteindre un rapport cétone en $C_4$-$C_6$/acétone de (50-50).

Selon une autre caractéristique additionnelle de la présente invention, après l'extraction liquide-liquide, on

2

EP 0 330 567 B1

sépare la phase organique à laquelle on ajoute à nouveau du sulfate d'ammonium. On récupère ensuite l'extrait par mise à sec de la phase cétonique. Cette mise à sec est obtenue de façon classique par exemple par concentration, puis par séchage sous vide.

Selon une autre caractéristique du procédé de l'invention, l'extrait sec peut être repris par un alcool, éventuellement aqueux, puis on élimine la fraction insoluble par filtration, et l'on récupère l'extrait final par remise à sec du filtrat alcoolique.

L'extraction primaire du broyat de feuilles de Ginkgo biloba est réalisée à l'aide d'un solvant cétonique aqueux. Selon une variante avantageuse du procédé de l'invention, l'extraction du broyat est obtenue à l'aide d'un mélange acétone-eau correspondant à des proportions comprises entre (80-20) et (60-40), de préférence de l'ordre de (70-30), à une température de l'ordre de 50 à 60°C.

L'objet de la présente invention s'étend également aux extraits de feuilles de Ginkgo biloba obtenus par la mise en oeuvre du procédé décrit ci-dessus, et en particulier aux extraits destinés à l'administration par voie orale et par voie injectable. Tel que cela apparaîtra dans la suite de la description, ces extraits, obtenus conformément au procédé de l'invention, présentent un très faible titre en proanthocyanidines, mais conservent en revanche une teneur appropriée en principes actifs recherchés. Dans la pratique, on obtient aisément des extraits présentant des titres acceptables en proanthocyanidines.

D'autres caractéristiques et avantages du procédé selon l'invention apparaîtront à la lecture de la description détaillée faite ci-après, en référence à un exemple de mise en oeuvre particulier.

EXEMPLE

Deux fois 800 g de feuilles vertes de Ginkgo biloba broyées sont extraits à contre-courant par deux fois 13,5 litres d'un mélange acétone-eau (70-30). Cette première extraction est conduite à une température comprise entre environ 50 et 60°C. Une température d'environ 55°C a été utilisée de façon tout à fait satisfaisante dans la pratique.

Après séparation du marc par filtration, les jus sont concentrés, sous pression réduite, jusqu'à un volume d'environ 1,9 litre, puis on procède à une séparation du précipité par décantation puis filtration.

La concentration sous vide suivie de la séparation du précipité par décantation et filtration constitue une première étape de purification du procédé selon l'invention, au cours de laquelle on élimine les biflavonoïdes et autres substances hydrophobes.

Le filtrat est ensuite additionné d'ammoniaque de façon à ajuster le pH à environ 9. On obtient ainsi une précipitation des proanthocyanidines. La fraction insoluble qui précipite est alors filtrée en vue d'éliminer les proanthocyanidines.

La basification du filtrat suivie de la filtration des proanthocyanidines constitue la seconde étape essentielle de purification du procédé selon l'invention.

On ajuste ensuite le pH du filtrat par acidification à l'aide d'acide sulfurique pour ramener le pH du filtrat jusqu'à une valeur voisine de 2. Le filtrat est alors extrait par environ 1,250 litre d'un mélange butanone/acétone (70/30) en présence d'environ 650 g de sulfate d'ammonium.

La phase organique obtenue au cours de cette extraction liquide-liquide est alors additionnée d'environ 200 g de sulfate d'ammonium supplémentaire, puis filtrée, concentrée et mise à sec sous pression réduite.

Cet extrait sec est alors repris dans 8 volumes d'éthanol aqueux, puis lavé par 2 volumes additionnels d'éthanol aqueux. La suspension ainsi obtenue est alors filtrée de manière à en éliminer la fraction insoluble. On récupère ainsi l'extrait final par concentration et mise à sec sous pression réduite, du filtrat alcoolique. Le rendement en poids est de 90‰.

L'analyse de l'extrait ainsi obtenu donne la titration suivante :

| Proanthocyanidines | 6,46 % |
| Glucosides flavoniques | 25,5 % |
| Quercétine + kaempférol | 6,6 % |

A titre de comparaison, un aliquote du concentrat aqueux clarifié, issu de la première étape de purification, a été traité selon un procédé habituel, c'est-à-dire sans précipitation des proanthocyanidines par basification du filtrat. Cet extrait de Ginkgo biloba titre :

3

| Proanthocyanidines | 35,2 % |
|---|---|
| Glucosides flavoniques | 24,5 % |
| Quercétine + kaempférol | 6,3 % |

Différentes expérimentations ont été conduites en modifiant la nature de l'agent de basification du filtrat, nécessaire à la précipitation des proanthocyanidines lors de la seconde étape de purification du procédé selon l'invention. A titre d'exemple, on indiquera ci-après dans le tableau I la composition des différents extraits tous obtenus à partir de lots de feuilles vertes de même qualité.

## TABLEAU I

| Elimination des proantho- cyanidines | Proantho- cyanidines % en poids | Glucosides flavoniques % en poids | Quercétine + kaempférol % en poids |
|---|---|---|---|
| Absence de basification | 35,2 | 24,5 | 6,3 |
| Ammoniaque | 6,46 | 25,5 | 6,6 |
| Hydroxyde de calcium | 1,2 | 21,3 | 5,3 |
| Soude | 16,2 | 22,8 | 6,4 |
| Diéthylamine | 7,0 | 25,5 | 7,3 |

Plusieurs extraits de feuilles de Ginkgo biloba présentant des teneurs différentes en proanthocyanidines ont fait l'objet d'une étude de toxicité aiguë chez la souris, par voie injectable.

L'administration a été effectuée sur des lots de 10 souris. L'extrait est mis en solution dans l'acétamide à 10% dans l'eau et administré sous le volume de 50 ml/kg.

Les résultats, relevés à T + 7 jours, sont consignés dans le tableau II ci-dessous.

## TABLEAU II

| PROANTHOCYANIDINES DANS L'EXTRAIT % en poids | $DL_{50}$ |
|---|---|
| 5,4 % | env. 1500 mg/kg |
| 18,5 % | env. 800 mg/kg |
| 30 % | env. 600 mg/kg |
| 59,6 % | env. 300 mg/kg |

**Revendications**

1. Procédé d'obtention d'un extrait de feuilles de Ginkgo biloba, caractérisé en ce que l'on effectue les opérations successives suivantes :
   – broyage des feuilles de Ginkgo biloba,
   – extraction du broyat de feuilles de Ginkgo biloba à l'aide d'un solvant cétonique aqueux,
   – concentration des jus d'extraction afin de précipiter les biflavonoïdes et les substances hydrophobes,
   – filtration du concentrat aqueux,
   – basification du filtrat pour faire précipiter les proanthocyanidines,
   – élimination par filtration de la fraction insoluble contenant les proanthocyanidines,
   – acidification du filtrat,
   – extraction liquide-liquide du filtrat par une cétone en $C_4$-$C_6$ en présence de sulfate d'ammonium, et
   – récupération de l'extrait par mise à sec de la phase cétonique.

2. Procédé selon la revendication 1, caractérisé en ce que la basification du filtrat est réalisée jusqu'à obtention d'un pH supérieur à 8.

3. Procédé selon l'une des revendications 1 et 2, caractérisé en ce que la précipitation des proanthocyanidines est obtenue par basification du filtrat au moyen d'hydroxydes de métaux alcalins ou alcalino-terreux, d'ammoniaque, d'ammoniac, ou d'une amine, notamment d'une alkylamine.

4. Procédé selon l'une des revendications 1 à 3, caractérisé en ce que l'acidification du filtrat, après élimination de leur fraction insoluble contenant les proanthocyanidines, est réalisée au moyen d'acide sulfurique.

5. Procédé selon la revendication 4, caractérisé en ce que ladite acidification est réalisée jusqu'à abaissement du pH à une valeur pouvant être voisine de 1.

6. Procédé selon l'une des revendications 1 à 5, caractérisé en ce que, après l'acidification, le filtrat est soumis à une extraction liquide-liquide par la méthyléthylcétone en présence de sulfate d'ammonium.

7. Procédé selon l'une des revendications 1 à 5, caractérisé en ce que, après acidification, le filtrat est soumis à une extraction liquide-liquide par la méthylisobutylcétone en présence de sulfate d'ammonium.

8. Procédé selon l'une des revendications 1 à 7, caractérisé en ce que la cétone en $C_4$-$C_6$ d'extraction liquide-liquide est utilisée en mélange avec de l'acétone.

9. Procédé selon la revendication 8, caractérisé en ce que le rapport cétone $C_4$-$C_6$/acétone peut contenir jusqu'à environ 50% en volume d'acétone.

10. Procédé selon l'une des revendications 1 à 9, caractérisé en ce que, après l'extraction liquide-liquide, on sépare la phase organique à laquelle on ajoute du sulfate d'ammonium.

11. Procédé selon l'une des revendications 1 à 10, caractérisé en ce que la mise à sec de la phase cétonique est réalisée par concentration, puis séchage sous vide.

12. Procédé selon l'une des revendications 1 à 11, caractérisé en ce que l'extrait sec obtenu à partir de la phase cétonique, est repris par un alcool, éventuellement aqueux, puis on élimine la fraction insoluble par filtration, et l'on récupère l'extrait final par mise à sec du filtrat alcoolique.

13. Procédé selon l'une des revendications 1 à 12, caractérisé en ce que l'extraction du broyat de feuilles de Ginkgo biloba est réalisée à l'aide d'un mélange acétone-eau compris entre (80-20) et (60-40), de préférence voisin de (70-30), à une température de l'ordre de 50 à 60°C.

14. Extrait de feuilles de Ginkgo biloba destiné à l'administration par voies orale et injectable, obtenu par la mise en oeuvre du procédé selon l'une des revendications 1 à 13, caractérisé en ce qu'il présente une teneur appropriée en principes actifs et une faible teneur en proanthocyanidines.

**Patentansprüche**

1. Verfahren zur Herstellung eines Extraktes der Blätter von Ginkgo biloba, dadurch gekennzeichnet, daß man nacheinander folgende Arbeitsgänge durchführt :
— Zerkleinern der Blätter von Ginkgo biloba,
— Extrahieren der zerkleinerten Blätter von Ginkgo biloba mit einem wäßrigen Keton-Lösungsmittel,
— Konzentrieren der Extraktionssäfte um die Biflavonoide und die hydrophoben Substanzen auszufällen,
— Filtrieren des wäßrigen Konzentrats,
— Basischmachen des Filtrats, um die Proanthocyanidine auszufällen,
— Entfernen der die Proanthocyanidine enthaltenden unlöslichen Fraktion durch Filtrieren,
— Ansäuern des Filtrats,
— Flüssig-Flüssig-Extraktion des Filtrats mit einem $C_4$-$C_6$-Keton in Anwesenheit von Ammoniumsulfat, und
— Wiedergewinnung des Extrakts durch Eintrocknen der Ketonphase.

2. Verfahren nach Anspruch 1, dadurch gekennzeichnet, daß das Basischmachen des Filtrats bis zur Erzielung eines pH-Werts über 8 durchgeführt wird.

3. Verfahren nach einem der Ansprüche 1 und 2, dadurch gekennzeichnet, daß die Ausfällung der Proanthocyanidine erzielt wird durch Basischmachen des Filtrats mittels Alkalimetall- oder Erdalkalimetall-Hydroxiden, Ammoniumhydroxid, Ammoniak oder einem Amin, insbesondere einem Alkylamin.

4. Verfahren nach einem der Ansprüche 1 bis 3, dadurch gekennzeichnet, daß das Ansäuern des Filtrats nach dem Entfernen der die Proanthocyanidine enthaltenden unlöslichen Fraktion mittels Schwefelsäure durchgeführt wird.

5. Verfahren nach Anspruch 4, dadurch gekennzeichnet, daß das Ansäuern bis zu einer Absenkung des pH-Werts auf einen Wert erfolgt, der nahe 1 liegen kann.

6. Verfahren nach einem der Ansprüche 1 bis 5, dadurch gekennzeichnet, daß das Filtrat nach dem Ansäuern einer Flüssig-Flüssig-Extraktion mit Methylethylketon in Anwesenheit von Ammoniumsulfat unterzogen wird.

7. Verfahren nach einem der Ansprüche 1 bis 5, dadurch gekennzeichnet, daß das Filtrat nach dem Ansäuern einer Flüssig-Flüssig-Extraktion mit Methylisobutylketon in Anwesenheit von Ammoniumsulfat unterzogen wird.

8. Verfahren nach einem der Ansprüche 1 bis 7, dadurch gekennzeichnet, daß das $C_4$-$C_6$-Keton für die Flüssig-Flüssig-Extraktion im Gemisch mit Aceton verwendet wird.

9. Verfahren nach Anspruch 8, dadurch gekennzeichnet, daß das Verhältnis $C_4$-$C_6$-Keton/Aceton bis zu etwa 50 Vol.-% Aceton enthalten kann.

10. Verfahren nach einem der Ansprüche 1 bis 9, dadurch gekennzeichnet, daß man nach der Flüssig-Flüssig-Extraktion die organische Phase abtrennt, der man Ammoniumsulfat zusetzt.

11. Verfahren nach einem der Ansprüche 1 bis 10, dadurch gekennzeichnet, daß das Eintrocknen der Ketonphase durch Konzentrieren und anschließendes Trocknen im Vakuum erfolgt.

12. Verfahren nach einem der Ansprüche 1 bis 11, dadurch gekennzeichnet, daß der aus der Ketonphase erhaltene trockene Extrakt mit einem gegebenenfalls wäßrigen Alkohol aufgenommen wird, worauf man die unlösliche Fraktion durch Filtration entfernt und den endgültigen Extrakt durch Eintrocknen des alkoholischen Filtrats gewinnt.

13. Verfahren nach einem der Ansprüche 1 bis 12, dadurch gekennzeichnet, daß die Extraktion der zerkleinerten Blätter von Ginkgo biloba mittels eines Gemischs Aceton-Wasser von (80-20) bis (60-40), bevorzugt von etwa (70-30), bei einer Temperatur in der Größenordnung von 50 bis 60°C durchgeführt wird.

14. Extrakt der Blätter von Ginkgo biloba, bestimmt zur Verabreichung auf oralem und injizierbarem Wege, erhalten durch Durchführung des Verfahrens nach einem der Ansprüche 1 bis 13, dadurch gekennzeichnet, daß er einen geeigneten Gehalt an aktiven Prinzipien und einen geringen Gehalt an Proanthocyanidinen aufweist.

## Claims

1. A method for obtaining an extract of Ginkgo biloba leaves, wherein the following successive operations are performed :

— grinding of the Ginkgo biloba leaves,

— extraction of the ground preparation of Ginkgo biloba leaves using an aqueous ketone solvent,

— concentration of the extraction liquors in order to precipitate the biflavonoids and the hydrophobic substances,

— filtration of the aqueous concentrate,

— alkalinization of the filtrate so as to precipitate the proanthocyanidins,

— removal by filtration of the insoluble fraction containing the proanthocyanidins,

— acidification of the filtrate,

— liquid-liquid extraction of the filtrate with a $C_4$-$C_6$ ketone in the presence of ammonium sulfate, and

— recovery of the extract by taking the ketone phase to dryness.

2. The process as claimed in claim 1, wherein the alkalinization of the filtrate is carried out until a pH above 8 is obtained.

3. The method as claimed in one of claims 1 and 2, wherein the precipitation of the proanthocyanidins is obtained by alkalinization of the filtrate by means of alkali metal or alkaline earth metal hydroxides, ammonia solution, ammonia gas or an amine, in particular an alkylamine.

4. Tie method as claimed in one of claims I to 3, wherein the acidification of the filtrate, after removal of the insoluble fraction containing the proanthocyanidins, is carried out by means of sulfuric acid.

5. The method as claimed in claim 4, wherein said acidification is carried out until the pH is lowered to a value which can be in the region of 1.

6. The method as claimed in one if claims 1 to 5, wherein, after acidification, the filtrate is subjected to a liquid-liquid extraction with methyl ethyl ketone in the presence of ammonium sulfate.

7. The method as claimed in one of claims 1 to 5, wherein, after acidification, the filtrate is subjected to a liquid-liquid extraction with methyl isobutyl ketone in the presence of ammonium sulfate.

8. The method as claimed in one of claims 1 to 7, wherein the $C_4$-$C_6$ ketone for liquid-liquid extraction is used mixed with acetone.

9. The method as claimed in claim 8, wherein the $C_4$-$C_6$ ketone/acetone ratio can contain up to approximately 50% by volume of acetone.

10. The method as claimed in one of claims 1 to 9, wherein, after the liquid-liquid extraction, the organic phase is separated off and ammonium sulfate is added thereto.

11. The method as claimed in one of claims 1 to 10, wherein, the taking of the ketone phase to dryness is carried out by concentration, followed by drying under vacuum.

12. The method as claimed in one of claims 1 to 11, wherein the dry extract obtained from the ketone phase is taken up with an alcohol, optionally aqueous, the insoluble fraction is then removed by filtration and the final extract is recovered by taking the alcoholic filtrate to dryness.

13. The method as claimed in one of claims 1 to 12, wherein the extraction of the ground preparation of Ginkgo biloba leaves is carried out using an acetone/ water mixture in proportions of between 80 : 20 and 60: 40, and preferably in the region of 70 : 30, at a temperature of the order of 50 to 60°C.

14. Extracts of Ginkgo biloba leaves obtained by carrying out the method as claimed in one of claims 1 to 13, and especially the extracts intended for administration orally and by injection, said extracts displaying a very low titer of proanthocyanidins and suitable content of the desired active principles.